# EUROPÄISCHE PATENTSCHRIFT

(11) **EP 3 570 720 B1**
(45) Veröffentlichungstag und Bekanntmachung des Hinweises auf die Patenterteilung: **03.08.2022**
(21) Anmeldenummer: 18702557.2
(22) Anmeldetag: 17.01.2018
(51) Int. Cl.: A61B 1/00

(54) **ENDOSKOP MIT EINEM ENDOSKOPKOPF UND EINEM AM ENDOSKOPKOPF EINSETZBAREN UND VON DIESEM LÖSBAREN ALBARRANHEBEL**
ENDOSCOPE WITH AN ENDOSCOPE HEAD AND AN ALBARRAN LEVER THAT CAN BE USED ON THE ENDOSCOPE HEAD AND CAN BE RELEASED FROM SAME
ENDOSCOPE COMPRENANT UNE TÊTE D'ENDOSCOPE ET UN LEVIER D'ALBARRAN POUVANT ÊTRE AGENCÉ SUR LA TÊTE D'ENDOSCOPE ET EN ÊTRE SÉPARÉ

(30) Priorität: 18.01.2017 DE 102017100867
(43) Veröffentlichungstag der Anmeldung: 27.11.2019
(73) Patentinhaber: Hoya Corporation, Tokyo 160-8347 (JP)
(72) Erfinder: DO, Anh Minh, 80689 München (DE)
(74) Vertreter: TBK
(86) Internationale Anmeldenummer: PCT/IB2018/000023
(87) Internationale Veröffentlichungsnummer: WO 2018/185546

(56) Entgegenhaltungen:
- DE-A1-102016 105 032
- DE-A1-102016 105 034
- DE-A1-102016 105 035
- US-A- 5 707 344

## Beschreibung

Die vorliegende Erfindung bezieht sich auf ein Endoskop mit einem Endoskopkopf mit einem Arbeitskanal zum Führen von Mikrowerkzeugen, und einem am Endoskopkopf einsetzbaren und von diesem lösbaren Albarranhebel mit einer Werkzeugleitfläche, an der ein durch den Arbeitskanal führbares Werkzeug in Kontakt treten kann, um in laterale Richtung des Endoskopkopfes abgelenkt zu werden.

Ein solcher Albarranhebel kann bei einem Endoskop zur Untersuchung z.B. der Speiseröhre oder auch des Duodenums, des Gallengangs, der Galle, des Bauchspeicheldrüsengangs, der Bauchspeicheldrüse etc., angewendet werden.

Ein solches Endoskop hat eine Optik (Beleuchtungseinrichtung und Kamera). Das Endoskop hat außerdem am Ausgang des Arbeitskanals den Albarranhebel, der durch Verschwenken eine gezielte Umlenkung der Werkzeuge ermöglicht, die durch den Arbeitskanal geschoben werden.

Nach der Anwendung des Endoskops wird dieses einer Aufbereitung unterzogen. Diese muss zuverlässig die Übertragung sämtlicher Keime oder Mikroorganismen wie Bakterien, Viren, Pilze, Würmer aber auch Sporen ausschließen. Bei der Aufbereitung wird zunächst das Endoskop manuell gereinigt, um organisches Material oder chemische Rückstände rückstandsfrei zu entfernen. Nach der Reinigung erfolgt eine maschinelle Desinfektion oder Sterilisation. Somit soll vermieden werden, dass Keime oder Mikroorganismen etc., mit denen ein Endoskop bei einem Einsatz des Endoskops in Kontakt gelangte, beim nächsten Einsatz auf den Patienten übertragen werden.

Beispielsweise offenbart die DE 196 27 016 C1 ein Endoskop mit einem Albarranhebel. Genauer gesagt hat das Endoskop einen vom Endoskop lösbaren Träger, in dem der Albarranhebel schwenkbar an einer im Träger abgestützten Achse angeordnet ist. Das Schwenken des Albarranhebels wird über ein Zugseil bewerkstelligt, das am Albarranhebel verankert ist und im Endoskop geführt wird.

Die DE 10 2016 105 034 A1 offenbart einen Endoskopkopf mit einem Albarranhebel, der in einem Albarranhebelanordnungsraum angeordnet ist. Der Albarranhebelanordnungsraum ist durch zwei Flankenabschnitte eines distalen Endoskopkopfes begrenzt. In einem der Flankenabschnitte ragt eine Albarranhebelschwenkwelle zu dem Albarranhebelanordnungsraum vor. Die Breite des Albarranhebel entspricht ungefähr der Breite des Albarranhebelanordnungsraumes.

Die vorliegende Erfindung hat die Aufgabe, ein Endoskop zu schaffen, bei dem noch besser vermieden wird, dass Keime, mit denen ein Endoskop in Kontakt gelangte, beim nächsten Einsatz auf den Patienten übertragen werden.

Diese Aufgabe ist durch ein Endoskop mit den Merkmalen von Anspruch 1 gelöst. Vorteilhafte Weiterbildungen sind Gegenstand der abhängigen Ansprüche.

Das erfindungsgemäße Endoskop hat einen Endoskopkopf mit einem Arbeitskanal zum Führen von Mikrowerkzeugen, und einen am Endoskopkopf einsetzbaren und von diesem lösbaren Albarranhebel mit einer Werkzeugleitfläche, an der ein durch den Arbeitskanal führbares Werkzeug in Kontakt treten kann, um in laterale Richtung des Endoskopkopfes abgelenkt zu werden. Ein Albarranhebelanordnungsraum ist von zwei sich in distaler Richtung erstreckenden Flankenabschnitten des Endoskopkopfes begrenzt. In einem der Flankenabschnitte ist eine Albarranhebelschwenkwelle gelagert, von der ein Wellenabschnitt zu dem Albarranhebelanordnungsraum mit einer Länge X vorragt. Der Albarranhebel weist ein Welleneinführloch zum Einführen des Wellenabschnittes hat und eine Breite a in einer Richtung, die parallel zum Welleneinführloch ist, auf, die lediglich um ein Maß eines Bewegungsspaltes geringer als die Breite des Albarranhebelanordnungsraumes in einer Richtung ist, die parallel zum Wellenabschnitt ist, wobei der Bewegungsspalt ein Spalt ist, der verhindert, dass der Albarranhebel beim Schwenken im Albarranhebelanordnungsraum an den Flankenabschnitten schleift.

Ein solches Endoskop bringt einen Aufbau mit sich, bei dem der Albarranhebel im Albarranhebelanordnungsraum betriebssicher geschwenkt werden kann, wobei der Albarranhebel selbst mit Leichtigkeit am Endoskop eingebaut und ausgebaut werden kann. Somit kann der Albarranhebel als Einmalverwendungsteil gestaltet werden. Nach der Anwendung wird der Albarranhebel entsorgt. Der nächste Einsatz des Endoskops erfolgt mit einem neuen (frischen) Albarranhebel. Eine Übertragung von Keimen durch einen Albarranhebel, der Hinterschnitte und eine komplexe Form haben kann, ist somit gar nicht mehr möglich.

Der Albarranhebel weist einen proximalen Basisabschnitt auf, in dem das Welleneinführloch ausgebildet ist. Der Basisabschnitt weist eine Breite b in einer Richtung, die parallel zum Welleneinführloch ist, auf, die der Breite a minus der Länge X entspricht. Dadurch wird ermöglicht, dass der Albarranhebel im Albarranhebelanordnungsraum auf einfache Weise eingesetzt werden kann, aber keine Befestigungselemente für das Befestigen des Albarranhebels am Endoskop benötigt werden. Somit wird das Endoskop kostengünstiger. Der in den Albarranhebelanordnungsraum vorragende Wellenabschnitt hat somit eine einfache Form, die keine Hinterschnitte etc. zum Ermöglichen eines Eingriffs oder eines Einsetzens von Befestigungselementen aufweist. Somit bietet auch der Wellenabschnitt weniger Anhaftmöglichkeiten für Keime und ist leichter und effektiver reinigbar. Der Bewegungsspalt kann zwischen dem Albarranhebel und dem jeweiligen Flankenabschnitt eine Größenordnung von ungefähr 0,01 mm bis 3/10 mm aufweisen. Vorzugsweise kann der Bewegungsspalt zwischen dem Albarranhebel und dem jeweiligen Flankenabschnitt eine Größenordnung von ungefähr 1/10 mm bis 2/10 mm aufweisen. Somit kann der Albarranhebel im Albarranhebelanordnungsraum zwischen den Flankenabschnitten sicher geführt werden.

Eine Kappe ist am Außenumfang auf den distalen Endabschnitt des Endoskopkopfes aufsetzbar, wobei im aufgesetzten Zustand das proximale Ende der Kappe proximal vom Albarranhebelanordnungsraum ist. Die Kappe schließt die noch offenen Seiten des Albarranhebelanordnungsraums und verhindert ein versehentliches Herausrutschen des Albarranhebels.

Die Kappe kann eine laterale Werkzeugöffnung an der Seite der Werkzeugleitfläche haben und kann an der distalen Seite geschlossen sein. Die Kappe behindert somit das Vorschieben eines Werkzeuges zur lateralen Seite nicht.

Die vorstehend erläuterten Aspekte der vorliegenden Erfindung können geeignet kombiniert werden.

### Kurzbeschreibung der Zeichnungen

Fig. 1 zeigt eine schematische Draufsicht auf einen Endoskopkopf mit einem Albarranhebel eines Ausführungsbeispiels der vorliegenden Erfindung, wobei der Albarranhebel gelöst ist.
Fig. 2 zeigt eine schematische Seitenansicht des Endoskopkopfes von Fig. 1.
Fig. 3 zeigt eine schematische Ansicht von der distalen Seite auf den Endoskopkopf mit dem Albarranhebel von Fig. 1, wobei der Albarranhebel dabei ist, angeordnet zu werden.
Fig. 4 zeigt eine schematische Seitenansicht des Endoskopkopfes in der Situation von Fig. 3.
Fig. 5 zeigt eine schematische Draufsicht auf den Endoskopkopf mit dem Albarranhebel von Fig. 1, wobei der Albarranhebel in der Arbeitsposition angeordnet ist.
Fig. 6 zeigt eine schematische Seitenansicht des Endoskopkopfes von Fig. 5.
Fig. 7 zeigt eine schematische Seitenansicht des Endoskopkopfes mit angeordnetem Albarranhebel.
Fig. 8 zeigt eine schematische Seitenansicht des Endoskopkopfes mit angeordnetem Albarranhebel, wobei eine Kappe aufgesetzt wird.
Fig. 9 zeigt eine schematische Draufsicht auf den Endoskopkopf mit dem Albarranhebel, wobei die Kappe aufgesetzt ist.
Fig. 10 zeigt eine schematische Seitenansicht des Endoskopkopfes von Fig. 9.

Nachstehend ist die vorliegende Erfindung detailliert unter Bezugnahme auf die Zeichnungen anhand eines Ausführungsbeispiels beschrieben.

### Ausführungsbeispiel

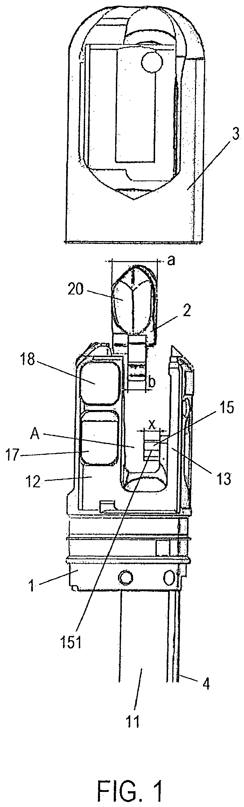

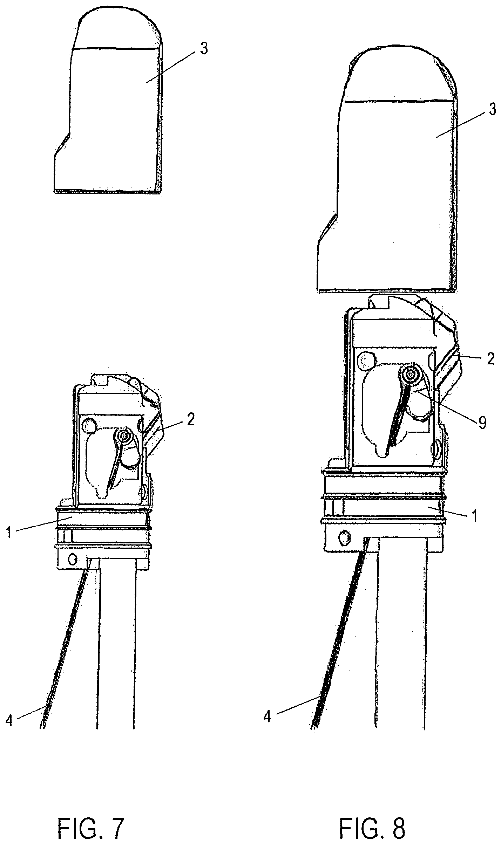

Nachstehend ist unter Bezugnahme auf die Figuren 1 bis 10 ein Ausführungsbeispiel der vorliegenden Erfindung beschrieben.

Das erfindungsgemäße Endoskop hat einen Endoskopkopf 1.

Zunächst ist unter Bezugnahme auf die Figuren der Endoskopkopf 1 beschrieben.

Der erfindungsgemäße Endoskopkopf 1 ist als zylindrischer Körper aufgebaut und weist einen Arbeitskanal 11 und einen nicht gezeigten Seilzugkanal auf, die sich jeweils entlang der Längsrichtung des Endoskopkopfes 1 und parallel zueinander erstrecken. Der Seilzugkanal führt ein Zugseil 4 zur Betätigung eines Albarranhebels 2. Der Arbeitskanal 11 führt Mikrowerkzeuge zur Untersuchung z.B. der Speiseröhre oder auch des Duodenums, des Gallengangs, der Galle, des Bauchspeicheldrüsengangs, der Bauchspeicheldrüse etc.

An der distalen Seite hat der Endoskopkopf 1 eine Optikerstreckung 12, an der eine Kamera 17 und eine Beleuchtungseinrichtung 18 in bekannter Weise vorgesehen sind, wobei diese Optikerstreckung 12 in Fig. 1 an der linken Seite in der Draufsicht gezeigt ist. Die Optikerstreckung 12 bildet einen Flankenabschnitt mit einer Kamera und Beleuchtung. Anders ausgedrückt bildet die Optikerstreckung 12 einen Gehäusevorsprung mit Kamera und Beleuchtung. Dieser Gehäusevorsprung ist seitlich neben einem nachstehend erörterten Albarranhebelanordnungsraum A angeordnet, um die Position und die Manipulationsbewegungen des Mikrowerkzeugs gut im Blickfeld der Kamera zu haben.

Der Arbeitskanal 11 endet in einem Abschnitt des Endoskopkopfes 1, der vom distalen Ende beabstandet ist, und bildet dort eine distale Ausgangsöffnung des Arbeitskanals.

Distal vom distalen Ausgang des Arbeitskanals 11 wird der Albarranhebel 2 angeordnet, der relativ zum Endoskopkopf 1 schwenken kann. Der Arbeitskanal 11 verläuft somit in distaler Richtung zum Albarranhebel 2 hin.

An der distalen Seite hat der Endoskopkopf 1 eine Albarranhebelabstützerstreckung 13, die in Fig. 1 an der rechten Seite der Draufsicht gezeigt ist. An der Albarranhebelabstützerstreckung 13 ist eine Schwenkwelle 15 so gelagert, dass sie sich zu einem Innenraum zwischen der Optikerstreckung 12 und der Albarranhebelabstützerstreckung 13 vorragt. Die Schwenkwelle 15 bildet einen Kraftübertragungsabschnitt, der eine Drehbewegungskraft auf den Albarranhebel 2 aufbringt. Die Schwenkwelle 15 ist quasi die Drehwelle des Albarranhebels 2. Die Albarranhebelabstützerstreckung 13 bildet somit einen Flankenabschnitt mit einer Albarranhebelschwenkwelle. Anders ausgedrückt bildet die Albarranhebelabstützerstreckung 13 einen Gehäusevorsprung mit Albarranhebelschwenkwelle.

Genauer gesagt wird die Drehbewegungskraft für den Albarranhebel 2 durch das Zugseil 4 aufgebracht, dessen Seilzugnippel an einem Ende eines Hebelelementes 9 gekoppelt ist. Das andere Ende des Hebelelementes 9 ist mit dem in der Albarranhebelabstützerstreckung 13 gelagerten Ende der Schwenkwelle 15 einstückig verbunden. Das entgegengesetzte Ende der Schwenkwelle 15 bildet einen aus der Albarranhebelabstützerstreckung 13 zur Optikerstreckung 12 vorragenden Wellenabschnitt 151 aus.

Der Albarranhebel 2 wird auf diesen in den Innenraum zwischen der Optikerstreckung 12 und der Albarranhebelabstützerstreckung 13 hineinragenden Wellenabschnitt 151 der Schwenkwelle 15 aufgesetzt. Der Wellenabschnitt 151 ist so positioniert, dass er der distalen Ausgangsöffnung des Arbeitskanals 11 gegenüberliegt. Der Innenraum zwischen der Optikerstreckung 12 und der Albarranhebelabstützerstreckung 13 bildet einen Albarranhebelanordnungsraum A. Der Albarranhebelanordnungsraum A ist somit von den beiden sich in distaler Richtung erstreckenden Flankenabschnitten 12 und 13 des Endoskopkopfes 1 begrenzt.

Die Schwenkwelle 15 hat somit ein Ende, das im Inneren der Albarranhebelabstützerstreckung 13 angeordnet ist. An dem zum Wellenabschnitt 151 entgegengesetzten Ende der Schwenkwelle 15 greift über das Hebelelement 9 das Zugseil 4 an. Anders ausgedrückt ist das distale Endes des Zugseils 4 mit dem zum Wellenabschnitt 151 entgegengesetzten Ende der Schwenkwelle 15 wirkverbunden. Ein in proximaler Richtung erfolgendes Ziehen des Zugseils 4 bewirkt somit ein Drehen der Schwenkwelle 15.

Das zum Wellenabschnitt 151 entgegengesetzte Ende der Schwenkwelle 15 ist gegenüber der Umgebung abgedichtet. Das Zugseil 4 ist im Seilzugkanal angeordnet. Der Seilzugkanal ist ebenfalls gegenüber der Umgebung abgedichtet. Somit hat das Zugseil 4 keinen Kontakt mit der Umgebung des Endoskopkopfes 1. Ein abgedichteter Raum, in dem das distale Ende des Zugseils 4, das Hebelelement 9 und das zum Wellenabschnitt 151 entgegengesetzte Ende der Schwenkwelle 15 angeordnet sind, ist in der Albarranhebelabstützerstreckung 13 vorgesehen. Dieser abgedichtete Raum ist lediglich zur proximalen Seite hin über den Seilzugkanal offen.

Der Wellenabschnitt 151 der Schwenkwelle 15 hat eine Form eines geraden Prismas mit einem Vieleck als Grundfläche in Längsrichtung der Schwenkwelle 15. Z.B. kann als Form des Wellenabschnittes 151 ein Dreikant oder Vierkant gewählt werden. Im vorliegenden Ausführungsbeispiel ist der Wellenabschnitt 151 in Vierkantform im Querschnitt ausgebildet. Der Wellenabschnitt 151 der Schwenkwelle 15 ragt mit einem Maß X in den Albarranhebelanordnungsraum A vor. Anders ausgedrückt hat der Wellenabschnitt 151 eine Länge X, siehe Fig. 1.

Nachstehend ist der Albarranhebel 2 beschrieben.

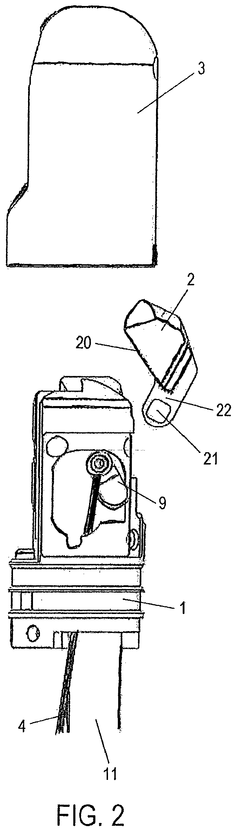

In Fig. 1 ist der Albarranhebel 2 von oben gezeigt, und in Fig. 2 ist der Albarranhebel 2 von der Seite gezeigt.

Der Albarranhebel 2 besitzt eine Werkzeugleitfläche 20, an der ein durch den Arbeitskanal 11 des Endoskopkopfes 1 führbares Werkzeug in Kontakt treten kann, um in laterale Richtung des Endoskopkopfes 1 (in Fig. 1 zum Betrachter hin) abgelenkt zu werden, so dass das Werkzeug z. B. in einen Gallengang eingeführt werden kann. Wenn der Albarranhebel 2 am Endoskopkopf 1 eingebaut ist, liegt die Werkzeugleitfläche 20 der distalen Endöffnung des Arbeitskanals 11 gegenüber.

An der proximalen Seite weist der Albarranhebel 2 einen Basisabschnitt 22 auf. Der Basisabschnitt 22 erstreckt sich in Einbaulage des Albarranhebels 2 in die proximale Richtung. Der Basisabschnitt 22 hat eine Breite mit einem Maß b, siehe Fig. 1. In Einbaulage des Albarranhebels 2 erstreckt sich die Breite des Basisabschnittes 22 parallel zur Albarranhebelschwenkwelle 15.

Im proximalen Bereich des Basisabschnittes 22 ist ein Welleneinführloch 21 als Durchgangsloch ausgebildet. Das Welleneinführloch 21 durchdringt den Basisabschnitt 22 in einer Richtung, die senkrecht zur Erstreckungsrichtung des Basisabschnittes 22 ist, wie dies aus Fig. 1 entnehmbar ist. Somit entspricht die Breite b des Basisabschnittes 22 der Länge des Welleneinführlochs 21.

Beim Anordnen des Albarranhebels 2 am Endoskopkopf 1 wird das Welleneinführloch 21 auf den Wellenabschnitt 151 gesetzt. Das Welleneinführloch 21 hat daher eine Innenform, die eine Gegenform zu der Form des Wellenabschnittes 151 bildet.

Die Form des Welleneinführlochs 21 muss dazu in der Lage sein, eine Drehung der Schwenkwelle 15 auf den Albarranhebel 2 zu übertragen. Im vorliegenden Ausführungsbeispiel ist das Welleneinführloch 21 in Vierkantform ausgebildet, wie dies in Fig. 2 gezeigt ist.

An der distalen Seite, also im Bereich der Werkzeugleitfläche 20 oder anders ausgedrückt im Bereich ohne den Basisabschnitt 22, hat der Albarranhebel 2 zwei parallele Seitenflächen. Diese parallelen Seitenflächen erstrecken sich in Einbaulage des Albarranhebels 2 in der Längsrichtung des Endoskops. In diesem distalen Bereich hat der Albarranhebel 2 eine Breite mit einem Maß a. Anders ausgedrückt ist sind die parallelen Seitenflächen im distalen Bereich des Albarranhebels 2 um das Maß a beabstandet.

In der vorliegenden Erfindung entspricht das Maß a ungefähr dem Maß b + dem Maß X.

Ferner ist das Maß a so gewählt, dass der Albarranhebel 2 in dem Albarranhebelanordnungsraum A mit einem geringfügigen Spiel drehbar ist. Die parallel zur Erstreckung der Schwenkwelle 15 gemessene Breite des Albarranhebelanordnungsraum A ist dabei etwas breiter als das Maß a. Somit ergibt sich bei eingebautem Albarranhebel 2 ein sehr kleiner Spalt zwischen der jeweiligen Seitenfläche im distalen Bereich des Albarranhebels 2 und der gegenüberliegenden Begrenzungsfläche des Albarranhebelanordnungsraums A, anders ausgedrückt der nach innen (zum Albarranhebelanordnungsraum A) weisenden Fläche der Optikerstreckung 12 und der Albarranhebelabstützerstreckung 13, d.h. des jeweiligen Flankenabschnittes. In Einbaulage des Albarranhebels 2 ergibt sich dabei ein jeweiliger Bewegungsspalt zwischen dem Albarranhebel 2 und dem jeweiligen Flankenabschnitt 12, 13 in einer Größenordnung von ungefähr 0,01 mm bis 0,3 mm. Vorzugsweise kann dieser Bewegungsspalt in einer Größenordnung von ungefähr 0,1 mm bis 0,2 mm sein. Dieser Bewegungsspalt verhindert, dass der Albarranhebel 2 beim Schwenken im Albarranhebelanordnungsraum A an den nach innen weisenden Flächen der Optikerstreckung 12 und der Albarranhebelabstützerstreckung 13 schleift.

Die Bestandteile des Albarranhebel 2 sind aus Kunststoff hergestellt. Der Albarranhebel 2 kann aus Kunststoff beispielsweise mittels 3D-Drucker oder Spritzformen hergestellt. Durch die Herstellung mittels 3D-Drucker oder Spritzformen kann der Albaranhebel genau aber noch unter geringen Kosten hergestellt werden. Weitere geeignete Herstellverfahren können aufgegriffen werden, solange diese eine genaue und kostengünstige Produktion erlauben.

Das erfindungsgemäße Endoskop hat des Weiteren eine auf den Endoskopkopf 1 aufsetzbare Kappe 3. Die Kappe 3 ist ein becherförmiger Körper, der als mit einem Boden versehener Zylinder ausgebildet ist. Die Kappe 3 hat eine Öffnung an der proximalen Seite, d.h. die in Fig. 1 nach unten weisende Seite. Über diese Öffnung wird die Kappe 3 auf den Außenumfang des distalen Endabschnittes des Endoskopkopfes 1 gesetzt. Die Kappe 3 hat eine Eingriffseinrichtung, mit der sie am Außenumfang des distalen Endabschnittes des Endoskopkopfes 1 in Eingriff gelangen kann, um einen festen Sitz der Kappe 3 am Endoskopkopf 1 zu gewährleisten. Z.B. ist die Eingriffseinrichtung in der Form einer Nase, eines Vorsprungs oder Hakens etc. vorgesehen, die im proximalen Bereich der Kappe 3 an der Innenumfangsfläche ausgebildet ist und mit einem entsprechenden Gegenelement (Nut etc.) am Außenumfang des Endoskopkopfes 1 in Eingriff gelangt.

Im aufgesetzten Zustand ist das proximale Ende der Kappe 3 proximal vom Albarranhebelanordnungsraum A. Die Kappe 3 ist an der distalen Seite geschlossen.

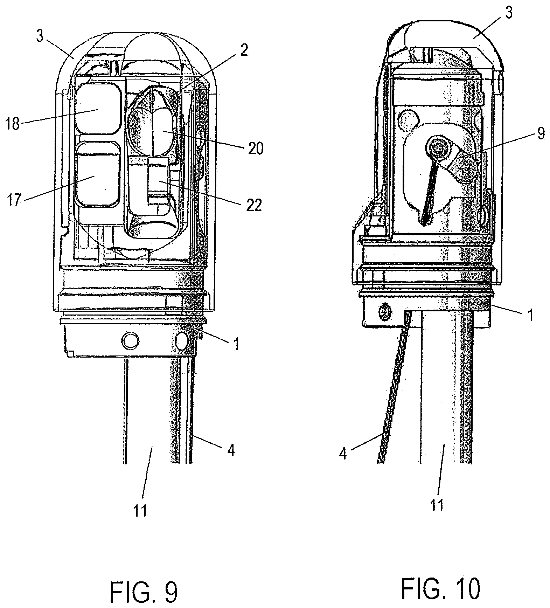

Die Kappe 3 hat eine laterale Werkzeugöffnung an der Seite der Werkzeugleitfläche 20, siehe Fig. 9. Die laterale Werkzeugöffnung legt nicht nur die Werkzeugleitfläche 20, sondern zumindest auch die Kamera 17 und die Beleuchtungseinrichtung 18 frei. Somit wird durch die laterale Werkzeugöffnung das seitliche Ausgeben des Werkzeugs, das Beleuchten durch die Beleuchtungseinrichtung 18 und das Aufnehmen eines Bildes durch die Kamera 17 ermöglicht.

Die Kappe 3 ist ebenfalls aus Kunststoff hergestellt. Die Kappe 3 kann aus Kunststoff beispielsweise mittels 3D-Drucker oder Spritzformen hergestellt. Durch die Herstellung mittels 3D-Drucker oder Spritzformen kann die Kappe 3 genau aber noch unter geringen Kosten hergestellt werden. Weitere geeignete Herstellverfahren können aufgegriffen werden, solange diese eine genaue und kostengünstige Produktion erlauben.

### Funktion der Erfindung

Das erfindungsgemäße Endoskop wird wie folgt angewendet.

Es wird ein neues Endoskop oder ein gereinigtes und sterilisiertes Endoskop bereitgestellt. Dieses Endoskop hat noch keinen Albarranhebel.

Der Albarranhebel 2 kann als separate steril verpackte Baugruppe vorgesehen werden, auf die ein Anwender des Endoskops zugreift. Der Anwender öffnet die Verpackung des Albarranhebels 2 und entnimmt den Albarranhebel 2.

Die bauliche Ausführung von Endoskopkopf 1 und Albarranhebel 2 ist so, dass der Albarranhebel 2 nicht in einer Einbaustellung, d.h. bei in proximaler Richtung weisendem Basisabschnitt 22, auf den Wellenabschnitt 151 gesetzt werden kann. Der Albarranhebel 2 kann zwar mit in proximaler Richtung weisendem Basisabschnitt 22 in den Albarranhebelanordnungsraum A eingeführt werden, es würde aber der der Außenumfang des proximalen Bereiches des Basisabschnittes 22 am Außenumfang des Wellenabschnittes 151 anstoßen, ohne dass das Welleneinführloch 21 auf den Wellenabschnitt 151 gesetzt werden kann. Die Maßbeziehung zwischen der Breite des distalen Abschnittes des Albarranhebels 2 und der Breite des Albarranhebelanordnungsraums A gestattet keine nennenswerte Schrägstellung des Albarranhebels 2 im Albarranhebelanordnungsraum A.

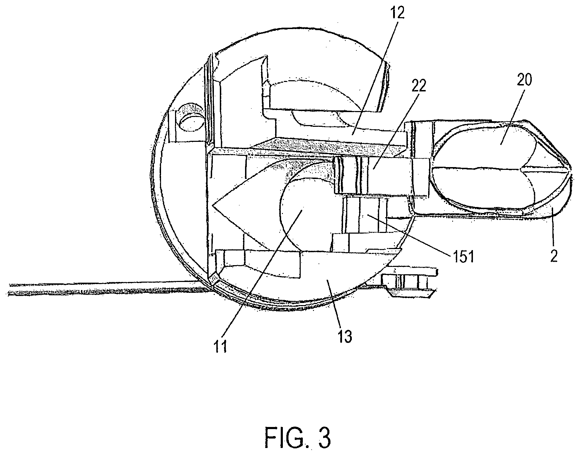

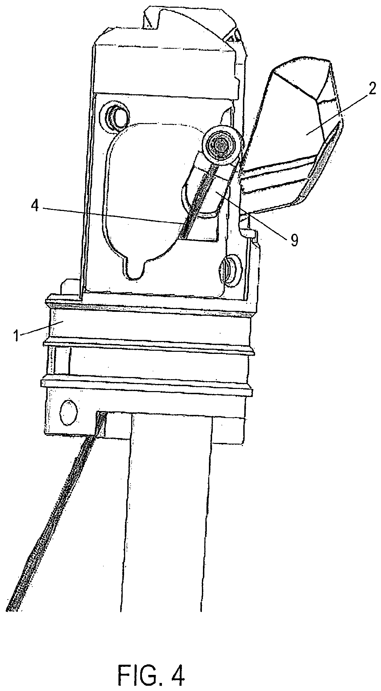

Daher muss der Albarranhebel 2 für den Einbau so gedreht werden, dass er ungefähr senkrecht zur Erstreckungsrichtung des Endoskopkopfes 1 ist, wobei sein proximaler Basisabschnitt 22 in den Albarranhebelanordnungsraum A weist. Senkrecht zur Erstreckungsrichtung des Endoskopkopfes 1 bedeutet hierbei ungefähr parallel zu der Richtung, in die die Kamera 17 und die Beleuchtungseinrichtung 18 weisen. In dieser Stellung des Albarranhebels 2 kann der Basisabschnitt 22 mit der Breite b zwischen dem Wellenabschnitt 151 und der nach innen (zum Albarranhebelanordnungsraum A) weisenden Fläche der Optikerstreckung 12 eingeführt werden, siehe Fig. 3 und Fig. 4. Der Basisabschnitt 22 mit der Breite b passt genau zwischen dem Wellenabschnitt 151 und der nach innen weisenden Fläche der Optikerstreckung 12 hinein. Der Seilzug 4 ist dabei so betätigt, dass die Albarranhebelschwenkwelle 15 die geeignete Drehlage hat.

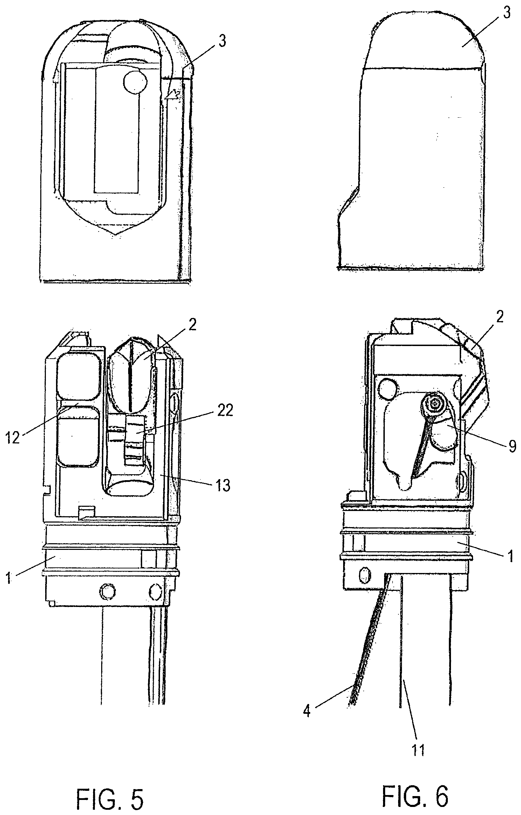

Nun kann der Albarranhebel 2 eingesetzt werden, indem der Basisabschnitt 22 so seitlich (in Fig. 3 nach unten) bewegt wird, dass der Wellenabschnitt 151 in das Welleneinführloch 21 eingeschoben wird, wobei dann der Albarranhebel 2 in die Ausgangsstellung geschwenkt werden kann, siehe Fig. 5.

Nun ist der Albarranhebel 2 im Albarranhebelanordnungsraum A schwenkbar. Aufgrund des zuvor festgelegten Bewegungsspaltes wird der Albarranhebel 2 an den Seitenwänden des Albarranhebelanordnungsraums A geführt, aber der Albarranhebel 2 schleift beim Schwenken nicht an den Seitenwänden des Albarranhebelanordnungsraums A.

Nun wird die Kappe 3 auf den Außenumfang des Endoskopkopfes 1 gesetzt. Dabei bildet die Kappe 3 eine zusätzliche Sicherung, die ein versehentliches Entfernen (Herausrutschen) des Albarranhebels 2 verhindert.

### Wirkungen der Erfindung

Der Albarranhebel 2 kann als separate steril verpackte Baugruppe vorgesehen werden, auf die ein Bediener des Endoskops zugreift.

Der Albarranhebel 2 ist leicht am Endoskopkopf 1 montierbar und demontierbar.

Der Albarranhebel 2 geht mit dem Wellenabschnitt 151 der Schwenkwelle 15 eine formschlüssige Verbindung ein, durch die die Kraft zum Schwenken des Albarranhebels 2 vom Endoskopkopf 1 zum Albarranhebel 2 sicher übertragbar ist.

Die Erfindung schafft einen einfachen und kostengünstigen Albarranhebel, der vom Zugseil 4 räumlich getrennt ist. Ein Albarranhebel bietet aufgrund seines geometrischen Aufbaus viele Hinterschnitte, an denen sich beim Gebrauch Keime etc. ansetzen können, die auch bei einem intensiven Reinigen und Sterilisieren möglicherweise am Albarranhebel verbleiben könnten. Der erfindungsgemäße Albarranhebel 2 kann aber nach einmaligem Gebrauch entsorgt werden. Somit bildet der erfindungsgemäße Albarranhebel 2 die Möglichkeit, dass ein Übertragen von Keimen etc., mit denen ein Endoskop bei einem Einsatz des Endoskops in Kontakt gelangte, beim nächsten Einsatz auf den nächsten Patienten vermieden wird.

Im Endoskopkopf ist der Seilzugkanal abgedichtet, wobei das Zugseil gegenüber der Umgebung vollständig abgedichtet ist. Das Abdichten des Seilzugkanals und des Seilzugs ist wasserdicht. Somit wird vermieden, dass Keime in den Seilzugkanal eindringen können oder mit dem Zugseil in Kontakt gelangen können.

Der Albarranhebel 2 kann an bisherigen Endoskopen angewendet werden, die einen entsprechenden Wellenabschnitt 151 haben.

Aufgrund der zuvor festgelegten Maße a, b, X und der Breite des Albarranhebelanordnungsraums A kann der Albarranhebel 2 am Endoskopkopf 1 ohne Hilfsmittel und ohne Befestigungsmittel wie z.B. Schrauben etc. befestigt werden, aber dennoch in einfacher Weise nach der Anwendung wieder vom Endoskopkopf 1 entfernt werden.

### Alternativen

Die geometrische Form des Wellenabschnittes 151 und des Welleneinführloches 21 ist nicht beschränkt, solange ein Formschluss zwischen beiden ermöglicht wird.

Der Eingriff der Kappe 3 am Außenumfang des Endoskopkopfes 1 unterliegt keinen Einschränkungen. Z.B. könnte die Kappe 3 am Außenumfang des Endoskopkopfes 1 aufgeschraubt werden.

Die Erfindung ist bei einem Duodenoskop anwendbar. Das Prinzip der Erfindung kann auch bei einem Ultraschallendoskop und bei jeder anderen Art an Endoskop angewendet werden.

In den Ausführungsbeispielen ist ein Arbeitskanal mit einem Albaranhebel am Ende des Arbeitskanals gezeigt. Die Erfindung kann auch bei Endoskopen angewendet werden, die mehrere Arbeitskanäle mit je einem Albaranhebel am Ende des jeweiligen Arbeitskanals aufweisen.

Die erläuterten Alternativen können kombiniert werden und können bei allen Ausführungsbeispielen angewendet werden.

### Bezugszeichenliste

- 1: Endoskopkopf
- 2: Albarranhebel
- 3: Kappe
- 4: Zugseil
- 9: Hebelelement
- 11: Arbeitskanal
- 12: Flankenabschnitt mit Kamera und Beleuchtung
- 13: Flankenabschnitt mit Albarranhebelschwenkwelle
- 15: Albarranhebelschwenkwelle
- 17: Kamera
- 18: Beleuchtungseinrichtung
- 20: Werkzeugleitfläche
- 21: Welleneinführloch
- 22: Basisabschnitt
- 151: Wellenabschnitt
- A: Albarranhebelanordnungsraum
- X: Länge des Wellenabschnittes
- a: Breite des Albarranhebels
- b: Breite des Basisabschnittes

## Patentansprüche

1. Endoskop mit
einem Endoskopkopf (1) mit einem Arbeitskanal (11) zum Führen von Mikrowerkzeugen, und
einem am Endoskopkopf (1) einsetzbaren und von diesem lösbaren Albarranhebel (2) mit einer Werkzeugleitfläche (20), an der ein durch den Arbeitskanal führbares Werkzeug in Kontakt treten kann, um in laterale Richtung des Endoskopkopfes abgelenkt zu werden,
wobei ein Albarranhebelanordnungsraum (A) von zwei sich in distaler Richtung erstreckenden Flankenabschnitten (12, 13) des Endoskopkopfes (1) begrenzt ist,
wobei in einem (13) der Flankenabschnitte (12, 13) eine Albarranhebelschwenkwelle (15) gelagert ist, von der ein Wellenabschnitt (151) zu dem Albarranhebelanordnungsraum (A) mit einer Länge (X) vorragt,
wobei der Albarranhebel (2) ein Welleneinführloch (21) zum Einführen des Wellenabschnittes (151) hat und eine Breite (a) in einer Richtung, die parallel zum Welleneinführloch (21) ist, aufweist, die lediglich um ein Maß eines Bewegungsspaltes geringer als die Breite des Albarranhebelanordnungsraumes (A) in einer Richtung ist, die parallel zum Wellenabschnitt (151) ist, wobei der Bewegungsspalt ein Spalt ist, der verhindert, dass der Albarranhebel (2) beim Schwenken im Albarranhebelanordnungsraum (A) an den Flankenabschnitten (12, 13) schleift, und
wobei der Albarranhebel (2) einen proximalen Basisabschnitt (22) aufweist, in dem das Welleneinführloch (21) ausgebildet ist,
**dadurch gekennzeichnet, dass**
der Basisabschnitt (22) eine Breite (b) in einer Richtung, die parallel zum Welleneinführloch (21) ist, aufweist, die der Breite (a) des Albarranhebels minus der Länge (X) des Wellenabschnittes entspricht.

2. Endoskop gemäß Anspruch 1, wobei
der Bewegungsspalt zwischen dem Albarranhebel (2) und dem jeweiligen Flankenabschnitt (12, 13) eine Größenordnung von ungefähr 0,01 mm bis 0,3 mm aufweist.

3. Endoskop gemäß einem der Ansprüche 1 oder 2, wobei
der Bewegungsspalt zwischen dem Albarranhebel (2) und dem jeweiligen Flankenabschnitt (12, 13) eine Größenordnung von vorzugsweise ungefähr 0,1 mm bis 0,2 mm aufweist.

4. Endoskop gemäß einem der Ansprüche 1 bis 3, mit
einer Kappe (3), die am Außenumfang auf den distalen Endabschnitt des Endoskopkopfes (1) aufsetzbar ist, wobei im aufgesetzten Zustand das proximale Ende der Kappe (3) proximal vom Albarranhebelanordnungsraum (A) ist.

5. Endoskop gemäß Anspruch 4, wobei
die Kappe (3) eine laterale Werkzeugöffnung an der Seite der Werkzeugleitfläche (20) hat und an der distalen Seite geschlossen ist.

## Claims

1. An endoscope having
an endoscope head (1) with a working channel (11) for guiding microtools, and
an Albarran lever (2) that is insertable on and detachable from the endoscope head (1), and having a tool guide surface (20) with which a tool that is guidable through the working channel may come into contact in order to be deflected in the lateral direction of the endoscope head,
wherein an Albarran lever arrangement space (A) is delimited by two flank sections (12, 13) of the endoscope head (1) that extend in the distal direction,
wherein an Albarran lever pivot shaft (15), from which a shaft section (151) protrudes with respect to the Albarran lever arrangement space (A) with a length (X), is supported in one (13) of the flank sections (12, 13),
wherein the Albarran lever (2) has a shaft insertion hole (21) for inserting the shaft section (151), and has a width (a) in a direction that is parallel to the shaft insertion hole (21), that is less than the width of the Albarran lever arrangement space (A), only by a dimension of a movement gap, in a direction parallel to the shaft section (151), the movement gap being a gap that prevents the Albarran lever (2) from rubbing against the flank sections (12, 13) while pivoting in the Albarran lever arrangement space (A), and
wherein the Albarran lever (2) has a proximal base section (22) in which the shaft insertion hole (21) is formed,
**characterized in that**
the base section (22) has a width (b), in a direction that is parallel to the shaft insertion hole (21), that corresponds to the width (a) of the Albarran lever minus the length (X) of the shaft section.

2. The endoscope according to claim 1, wherein the movement gap has a range of approximately 0.01 mm to 0.3 mm between the Albarran lever (2) and the particular flank section (12, 13) .

3. The endoscope according to any of claims 1 or 2, wherein the movement gap has a range of preferably approximately 0.1 mm to 0.2 mm between the Albarran lever (2) and the particular flank section (12, 13).

4. The endoscope according to any of claims 1 to 3, having a cap (3) that is mountable on the outer circumference, on the distal end section of the endoscope head (1), wherein in the mounted state the proximal end of the cap (3) is proximal from the Albarran lever arrangement space (A).

5. The endoscope according to claim 4, wherein
the cap (3) has a lateral tool opening on the side of the tool guide surface (20) and is closed on the distal side.

## Revendications

1. Endoscope avec
une tête d'endoscope (1) avec un canal de travail (11) pour guider des micro-instruments, et
un levier d'Albarran (2) pouvant être inséré sur la tête d'endoscope (1) et pouvant être détaché de celle-ci avec une surface d'acheminement d'instrument (20), sur laquelle un instrument pouvant être guidé à travers le canal de travail peut entrer en contact, afin d'être dévié dans la direction latérale de la tête d'endoscope,
dans lequel un espace de disposition de levier d'Albarran (A) est délimité par deux parties de flanc (12, 13) de la tête d'endoscope (1) s'étendant dans la direction distale,
dans lequel un arbre de pivotement de levier d'Albarran (15), duquel une partie d'arbre (151) fait saillie vers l'espace de disposition de levier d'Albarran (A) avec une longueur (X), est monté dans une (13) des parties de flanc (12, 13),
dans lequel le levier d'Albarran (2) possède un trou d'introduction d'arbre (21) pour l'introduction de la partie d'arbre (151) et présente une largeur (a) dans une direction qui est parallèle au trou d'introduction d'arbre (21) qui est simplement inférieure à la largeur de l'espace de disposition de levier d'Albarran (A) d'une valeur d'une fente de mouvement dans une direction qui est parallèle à la partie d'arbre (151), dans lequel la fente de mouvement est une fente qui empêche que le levier d'Albarran (2) lors du pivotement dans l'espace de disposition de levier d'Albarran (A) frotte sur les parties de flanc (12, 13), et
dans lequel le levier d'Albarran (2) présente une partie de base (22) proximale, dans laquelle le trou d'introduction d'arbre (21) est réalisé,
**caractérisé en ce que**
la partie de base (22) présente une largeur (b) dans une direction qui est parallèle au trou d'introduction d'arbre (21), qui correspond à la largeur (a) du levier d'Albarran moins la longueur (X) de la partie d'arbre.

2. Endoscope selon la revendication 1, dans lequel
la fente de mouvement entre le levier d'Albarran (2) et la partie de flanc (12, 13) respective présente un ordre de grandeur d'environ 0,01 mm à 0,3 mm.

3. Endoscope selon l'une quelconque des revendications 1 ou 2, dans lequel
la fente de mouvement entre le levier d'Albarran (2) et la partie de flanc (12, 13) respective présente un ordre de grandeur de préférence d'environ 0,1 mm à 0,2 mm.

4. Endoscope selon l'une quelconque des revendications 1 à 3, avec
un capuchon (3), qui peut être posé sur la périphérie extérieure sur la partie d'extrémité distale de la tête d'endoscope (1), dans lequel dans l'état posé l'extrémité proximale du capuchon (3) est proximale de l'espace de disposition de levier d'Albarran (A).

5. Endoscope selon la revendication 4, dans lequel
le capuchon (3) possède une ouverture d'instrument latérale sur la face de la surface d'acheminement d'instrument (20) et est fermé sur la face distale.
